(19) Europäisches Patentamt / European Patent Office / Office européen des brevets

(11) **EP 4 474 819 B1**

(12) **EUROPEAN PATENT SPECIFICATION**

(45) Date of publication and mention
of the grant of the patent:
**07.05.2025 Bulletin 2025/19**

(21) Application number: **24177714.3**

(22) Date of filing: **23.05.2024**

(51) International Patent Classification (IPC):
**G01N 33/38** (2006.01)

(52) Cooperative Patent Classification (CPC):
**G01N 33/383;** G01N 2223/079; G01N 2223/418;
G01N 2223/616

(54) **METHOD OF DETERMINING THE COMPOSITION OF CEMENT-LIME MORTAR**

VERFAHREN ZUR BESTIMMUNG DER ZUSAMMENSETZUNG VON ZEMENT-KALK-MÖRTEL

PROCÉDÉ POUR DÉTERMINER LA COMPOSITION DE MORTIER DE CIMENT ET CHAUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.06.2023 PL 44512123**

(43) Date of publication of application:
**11.12.2024 Bulletin 2024/50**

(73) Proprietor: **Instytut Techniki Budowlanej
00-611 Warszawa (PL)**

(72) Inventor: **Chylinski, Filip
Dziekanow Lesny (PL)**

(74) Representative: **AOMB Polska Sp. z.o.o.
ul. Rondo Ignacego Daszynskiego 1
00-843 Warsaw (PL)**

(56) References cited:
• QUARCIONI V A ET AL: "Optmization of calculation method for determination of composition of hardened mortars of Portland cement and hydrated lime made in laboratory", CONSTRUCTION AND BUILDING MATERIALS, vol. 20, no. 10, 27 September 2005 (2005-09-27), pages 1069 - 1078, XP027933755, ISSN: 0950-0618

• HE H ET AL: "The effect of grain size on Gmaxof a demolished structural concrete: A study through energy dispersive spectroscopy analysis and dynamic element testing", SOIL DYNAMICS AND EARTHQUAKE ENGINEERING, vol. 89, 17 August 2016 (2016-08-17), pages 208 - 218, XP029734643, ISSN: 0267-7261, DOI: 10.1016/J.SOILDYN.2016.08.011

• FREIRE MARIA TERESA ET AL: "Studies in ancient gypsum based plasters towards their repair: Mineralogy and microstructure", CONSTRUCTION AND BUILDING MATERIALS, vol. 196, 23 November 2018 (2018-11-23), pages 512 - 529, XP085574890, ISSN: 0950-0618, DOI: 10.1016/J.CONBUILDMAT.2018.11.037

• "Standard EN 196-2: 2013. Method of testing cement - Part 2: Chemical analysis of cement", 1 June 2013 (2013-06-01), XP009557420, Retrieved from the Internet <URL:https://www.nen.nl/nen-en-196-2-2013-en-184482>

## Description

### Field of the invention

The present invention relates to a method of determining the composition of masonry mortar. More specifically, the invention concerns the use of scanning microscopy with composition analysis in a SEM-EDS micro-area and the determination of the content of insoluble residue in hydrochloric acid in methods related to the determination of cement-lime mortar composition.

### State of the art

Masonry mortars are binders used to bond small-scale masonry units into a structure. Masonry mortar consists mainly of fine aggregate (usually sand), mineral binder and water. In accordance with the European standard PN-EN 998-2:2016-12 *Specification for mortar for masonry - Part 2: Masonry mortar,* masonry mortar is a mixture containing one or more types of mineral binders, aggregate and water and may also contain admixtures and/or additives. The types of mineral binders used can be divided into three main groups: lime, cement and cement-lime, in accordance with the standard PN-B-10104 *Requirements for masonry mortars of general use - Prescribed masonry mortars, mixed on building site.* In each of these groups, additional subgroups can be distinguished, characterized by additional characteristics of the binder belonging to a given subgroup, for example different types of lime, cements or active additives used. During repair works, it is necessary to use mortar with the same composition as the primary mortar and with similar values of strength parameters. The use of mortars with lower strength than the original one leads to a reduced load-bearing capacity of the entire masonry structure. In turn, the use of mortars with excessive strength for repair works may lead, in the event of the appearance of locally high loads, to the formation of cracks in masonry elements instead of their appearance in the joint, which is an unfavourable phenomenon due to the possibility of carrying out subsequent repairs.

Determining the composition of cement mortars and lime mortars does not usually pose major problems on account of only two unknown values, which are: the content of aggregate and the content of cement or lime, respectively. A problem arises when trying to determine the composition of cement-lime mortars, in which a mixture of cement and lime is used as a binder. The hydration products of both binder components in terms of elemental composition are similar and contain mainly calcium (Ca), silicon (Si) and aluminium (Al) in different proportions. Therefore, the results of examining the elemental composition of the entire mortar, for example by X-ray fluorescence spectroscopy (XRF), may not provide unambiguous information about the type of mortar. This is also due to the fact that Si, Ca, Al, which are the most abundantly present in the composition of the mortar, are also components of the aggregate.

Hardened cement-lime mortars contain similar crystalline components as portlandite, calcite, C-S-H phase and clinker relics, which make it difficult to quantify the content of components by X-ray diffraction (XRD). An additional problem in such identification is the use of sand from various deposits possessing diverse composition.

In connection with the above-mentioned facts, the need to develop a research method allowing one to determine the composition of cement-lime mortars arose. The method presented consists of two stages: determination of the cement/lime ratio in the binder and determination of the aggregate content in the mortar.

In the current state of the art, solutions for the application of the scanning microscopy technique with analysis in the SEM-EDS micro-area in the study of the composition of masonry mortar are known. In Klimek, B. "Badania historycznych zapraw z Baszty Gotyckiej w Lublinie., TEKA Komisji Architektury, Urbanistyki i Studiów Krajobrazowych Oddział Polskiej Akademii Nauk w Lublinie 2022, 18(3), pp. 18-29, analyses of the elemental composition on fracture samples in individual, selected points of the masonry mortar collected from the Gothic Tower in Lublin were presented. The document did not examine the type of masonry mortar. In addition, no research was carried out using the methodology consisting in the study of polished microsections with the analysis of the elemental composition of binder mass areas without taking into account aggregate grain and the subsequent establishment of the Si/Ca ratio. In addition, the authors did not determine the exact proportions of the ingredients used in the production of the mortar and did not use the Si/Ca ratio or reference mortars. In Słomka-Słupik B., et al., "Diagnostyczne badania wykwitów solnych na restaurowanych elewacjach Część 2 - badania zapraw i tynków.", Ochrona przed Korozją 2018, 61(2), pp. 43-47, the SEM-EDS technique was used to determine the elemental composition at selected points of the analysed fractures in the samples collected, including mortar samples. The document did not address the topic of identifying the type of masonry mortar used from among lime, cement-lime or cement mortar.

In the Wrocław University of Technology, Faculty of Architecture - Technical and Conservation Laboratory, "WYNIKI BADAŃ PROBEK ZAPRAW Badania w ramach projektu badawczego pn.: Cmentarz Salvatora - pierwsza nekropolia wroclawskich protestantów" Series: W1/ 430-3/2014, June 2014, the results of mortar samples examination were presented. The scope of the research also included microstructural studies using the SEM-EDS technique. The research presented in the document was limited to the observation of the microstructure of mortar fractures and point analysis of the elemental composition in selected sites of the areas observed. The microscopic observations presented are typical studies performed using SEM-EDS.

V. Quarzioni et al. in Construction and Building Materials 20 (2006), pages 1069-1078, disclose a chemical analysis of the composition of cement-lime mortars.

H. He et al. in Soil Dynamics and Earthquake Engineering 89 (2016), pages 208-218, disclose obtaining the Si/Ca ratio from SEM-EDS.

M. T. Freire et al. in Construction and Building Materials 196 (2019), pages 512-529, disclose preparing a sample by immersing it in epoxy, grinding it, then sputtering with gold.

## The subject matter of the invention

The subject matter of the invention is a method for determining the composition of cement-lime masonry mortar including the following stages:

> determination of the % atomic ratio of cement/lime in the binder, including:
>
>> mechanical collection of a mortar fragment, drying the sample from the stage i) at a temperature of at least 40 °C for at least 8 hours, then immersing the cooled sample in epoxy resin under vacuum, exposing the surface of the mortar cross-section by grinding and sputtering it with a conductive material selected from a group including gold, platinum, palladium, osmium, and carbon, with the sputtered layer being at least 1 nm thick;
>> determining the cement/lime ratio in the binder mass based on the Si/Ca content ratio, by microscopic examination of the sample from stage ii) using a scanning microscope with an accelerating voltage of not less than 10 kV and an aperture of not less than 1 $\mu$m
>
> determination of the aggregate content in the mortar:
>
>> cutting out a fragment of cement-lime mortar and drying it at a temperature not lower than 40 °C for at least 8 hours, followed by its grinding in entirety to obtain grains measuring less than 0.125 mm.
>> determination of the aggregate content in the cement-lime mortar on the basis of the content of insoluble residue in aqueous HCl and $Na_2CO_3$ solutions in the sample prepared in stage iv), in accordance with the standard PN-EN 196-2: 2013-11.
>
> in which the microscopic examination of the prepared sample consists in studying the binder mass alone in the sample, omitting the aggregate grain, with a SEM scanning electron microscope integrated with an ED X-ray energy dispersion spectrometer.

The subject matter of the invention presented is the stage of determining the composition of the binder mass by analysing the elemental composition with the SEM-EDS method only in the areas of binder mass, without taking into account the composition of the aggregate, and then determining the atomic ratio or mass ratio of Si/Ca. Based on the analyses carried out in this manner and a set of data from reference samples of a known composition, the content of lime in the binder mass is determined. The second stage is to determine aggregate content in the entire cement-lime mortar using the method of determining insoluble residue in HCl and $Na_2CO_3$. This is a standard test performed in accordance with the standard PN-EN 196-2:2013-11 *Method of testing cement 2: Chemical analysis of cement - Part.*

The method according to the invention, unlike other methods, allows for accurate determination of the composition of the cement-lime masonry mortar under study. Limiting the examination to the area of the binder mass alone, excluding the aggregate grain, has a positive effect on the accuracy of the results obtained. Such information may, among other things, assist in recreating mortar for fillings used in restoration works.

Brief description of the drawings

The subject matter of the invention is illustrated in its embodiments in the drawing, in which:

> Fig. 1. shows an example image of a sample of cement-lime mortar;
> Fig. 2. shows an analysis of the elemental composition of the binder mass area indicated in Fig. 1. with a white line;

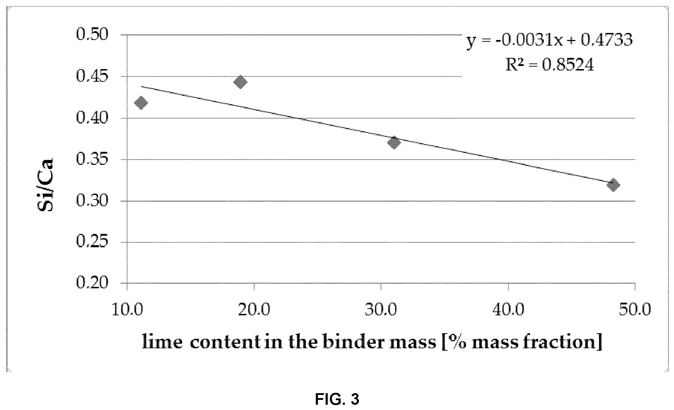

> Fig. 3. shows a graph of the dependence of Si/Ca on the lime/cement ratio in the binder mass.

## Embodiments of the invention

The test samples may be fragments of mortar collected from a masonry structure or another element, mechanically, for example by cutting out or drilling cores. After sampling, the samples are prepared for microscopic examinations and for testing the content of insoluble residue.

**Embodiment 1. Preparation of samples for microscopic examination.**

The cut-out fragment of the cement-lime mortar with a volume of 1cm$^3$ was dried at 40 °C for 8 hours; in the case of a larger sample, drying may proceed at a higher temperature and for at least 8 hours. Then, after cooling, it was immersed in epoxy resin under vacuum. After the epoxy resin had hardened, the sample was subjected to grinding and polishing in order to reveal an even and smooth cross-sectional surface of the mortar. Immediately before performing observation using a scanning microscope, the sample was sputtered with conductive material, for example gold, platinum, palladium, osmium or carbon. The thickness of the sputtered layer is at least 1 nm.

Embodiment 2. Preparation of samples for examining the content of insoluble residue.

The cut-out fragments of the cement-lime mortar with a mass of 10 g were dried at a temperature of 40 °C for 8 hours; in the case of a larger sample, drying may proceed at a higher temperature and for at least 8 hours. Then, they were ground in entirety to obtain grains measuring less than 0.125 mm.

**Embodiment 3. Examination of the sample prepared for microscopic examinations.**

The prepared mortar samples are examined using a scanning microscope, and the elemental composition of the binder mass alone is analysed, omitting aggregate grains. The analysis of the composition is limited only to the content of Ca, Si and Al. Examination is carried out in several randomly selected areas of the sample under study. It is crucial that all analyses are carried out with the use of the same equipment, at the same magnification and using the same exposure parameters, including accelerating voltage, aperture and working distance of the sample from the electron gun.

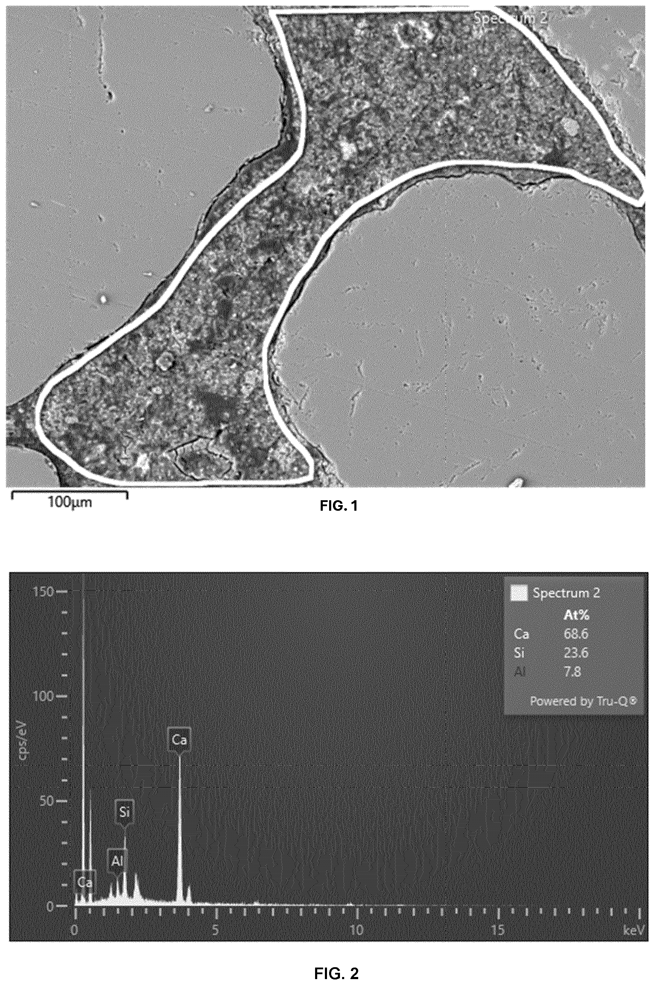

The prepared sample of cement mortar was subjected to microscopic examinations. The sample was analysed at a constant magnification of 500x, with accelerating voltage of 20 kV and an aperture of 120 $\mu$m. The minimum accelerating voltage is 10 kV and the minimum aperture is 1 $\mu$m. A sample image and the result of analysing the composition of the binder mass are shown in Fig. 1 and Fig. 2.

The examination of the binder mass composition was repeated in ten randomly selected areas of the sample, recording the content of Ca, Si, Al in atomic ratios. The values obtained were averaged obtaining the following content figures:

- Ca = 69.08%

- Si = 24.30%

- Al = 6.60%

- Si/Ca = 0.352.

On the basis of the microscopic results collected, the ratio of Si/Ca, Al/Ca and Al/Si content is determined, and it was found that the Si/Ca ratio is the most useful for determining the lime/cement ratio in the binder mass. Based on a series of reference analyses of cement-lime mortars of a known composition, a linear dependence of the value of the Si/Ca ratio on the content of lime in the binder mass was determined.

Studies of reference cement-lime mortars of known composition were carried out and the average content of Ca, Si, Al was determined for each of the mortars with the same observation parameters. The Si/Ca ratio was determined for each of the mortars. A linear dependence of the value of Si/Ca on the content of lime in the binder mass was determined. The graph of the dependence obtained is shown in Fig. 2.

The Si/Ca value of the mortar under study was 0.352. By using the function equation in Fig. 2. it is possible to determine the content of lime in the binder mass at 39.13%, with cement constituting the remaining part (60.87%) of it.

**Embodiment 4. Determination of the insoluble residue content.**

Determination of the aggregate content by determining insoluble residue in HCl and $Na_2CO_3$ is carried out according to the standards.

In accordance with the standard PN-EN 196-2: 2013-11, 1 g of the tested sample should be weighed out with an accuracy of 0.0001 g ($m_z$) and transferred to a beaker. Then demineralised water in the amount of 90 cm³ and concentrated hydrochloric acid in the amount of 10 cm³ are added. The whole mixture is heated to near-boiling temperature and maintained in it for 15 minutes, and then it is filtered using analytical filters. The sediment on the filter is repeatedly washed with hot demineralised water until the presence of chloride ions in the filtrates disappears. The filter with sediment is then transferred to a beaker and immersed in a solution of sodium carbonate at a concentration of 5% in an amount of 100 cm³. The whole liquid is brought to a boil and kept at this temperature for 30 minutes. Then the whole contents are filtered using a new filter and rinsed four times with hot demineralised water, and then with hot hydrochloric acid (1+19) until a pH value below 2 is obtained. The filter is rinsed at least ten times with hot demineralised water until the presence of chlorides in the filtrates has disappeared, checking the filtrates with a silver nitrate solution. The filter with the contents is transferred quantitatively to a previously calcined and weighed out crucible ($m_0$). It is then incinerated and heated at 950 °C. After cooling, the crucible is weighed ($m_k$) and the content of insoluble residue (N) is calculated from the formula:

$$N = \frac{m_k - m_0}{m_z} \times 100$$

wherein:

N - insoluble residue content [%],

$m_z$ - mass of the weighed-out amount of the cement-lime mortar [g],

$m_0$ - mass of an empty crucible [g],

$m_k$ - mass of the crucible with the residue after heating [g].

**Embodiment 5. Determination of aggregate content in cement-lime mortar.**

The content of aggregate in mortar is determined on the basis of the content of solids which are insoluble in HCl and $Na_2CO_3$. It can be assumed directly that the content of aggregate in the mortar is equal to the determined value of insoluble residue. However, such simplification carries an error in the order of several percentage points due to the fact that fine natural aggregate is not characterised by the value of insoluble residue equal to 100%, but most often approaches 95%, and the binder mass itself does not dissolve completely. In order to increase accuracy, a series of cement-lime mortars of a known composition could be tested using the same aggregate and the value of the correction factor could be determined on this basis.

The sample of cement-lime mortar was examined to determine the content of solids which are insoluble in HCl and $Na_2CO_3$, obtaining the value of 81.54%. On the basis of the determined dependence of the content of insoluble residue on the amount of aggregate in mortar for reference mortars, a correction factor of 1.073 was determined. After multiplying both values, the value of 87.49% was obtained, which describes the mass fraction of aggregate in the mortar. The remaining part (12.51%) is the binder mass.

**Embodiment 6. Determination of the composition of the mortar under study.**

On the basis of the analyses carried out, the content of aggregate in the mortar, the content of binder mass as well as the content of lime and cement in the binder mass were determined, which allows for determining the full composition of cement-lime mortar.

The analyses conducted allowed for determining the following composition values:

- aggregate content 87.49%,

- binder mass content 12.51%,

- lime content in the binder mass 39.13%,

- cement content in the binder mass 60.87%.

From the above data, it is possible to calculate:

- lime content in the mortar = 0.1251 x 39.13% = 4.90%

- cement content in the mortar = 0.1251 x 60.87% = 7.61%.

In summary, the composition of the mortar under study is as follows:

- aggregate 87.49%,

- cement 7.61%

- lime 4.90%.

**Claims**

1. A method for determining a composition of a cement-lime masonry mortar involving the following stages:

a) determination of the % atomic ratio of cement/lime in the binder, including:

i) mechanical collection of a mortar fragment,
ii) drying the sample from stage i) at a temperature not lower than 40 °C for at least 8 hours, then immersing the cooled sample in epoxy resin under vacuum, exposing the surface of the cross-section of the mortar by grinding and polishing, as well as sputtering it with a conductive material selected from a group including gold, platinum, palladium, osmium and carbon, with the thickness of the layer formed by sputtering being at least 1 nm;
iii) determining the cement/lime ratio in the binder mass based on the Si/Ca content ratio, by microscopic examination of the sample from stage ii) using a scanning microscope with an accelerating voltage of not less than 10 kV and an aperture of not less than 1 $\mu$m

b) determination of the aggregate content in the mortar:

iii) cutting out a fragment of cement-lime mortar and drying it at a temperature not lower than 40 °C for at least 8 hours, followed by its grinding in entirety to obtain grains measuring less than 0.125 mm.
iv) determination of the aggregate content in the cement-lime mortar on the basis of the quantity of insoluble residue in aqueous HCl and $Na_2CO_3$ solutions in the sample prepared in stage iv), in accordance with the standard PN-EN 196-2: 2013-11.

**characterized in that** the microscopic examination of the prepared sample is the examination of the binder mass in the sample alone, omitting the aggregate grain, with a SEM scanning electron microscope integrated with an EDS X-ray energy dispersion spectrometer.

**Patentansprüche**

1. Verfahren zum Bestimmen einer Zusammensetzung eines Zement-Kalk-Mauermörtels, das die folgenden Schritte beinhaltet:

a) Bestimmung des Atomverhältnisses von Zement/Kalk im Bindemittel in %, einschließlich:

i) mechanischer Entnahme eines Mörtelfragments,
ii) Trocknen der Probe aus Schritt i) bei einer Temperatur von nicht weniger als 40 °C für mindestens 8 Stunden, dann Eintauchen der abgekühlten Probe in Epoxidharz unter Vakuum, Freilegen der Oberfläche des Querschnitts des Mörtels durch Schleifen und Polieren sowie dessen Sputtern mit einem leitfähigen Material, ausgewählt aus einer Gruppe, die Gold, Platin, Palladium, Osmium und Kohlenstoff einschließt, wobei die Dicke der durch Sputtern gebildeten Schicht mindestens 1 nm beträgt;
iii) Bestimmen des Zement/Kalk-Verhältnisses in der Bindemittelmasse basierend auf dem Si/Ca-Gehaltsverhältnis durch mikroskopische Untersuchung der Probe aus Stufe ii) unter Verwendung eines Rastermikroskops mit einer Beschleunigungsspannung von nicht weniger als 10 kV und einer Blende von nicht weniger als 1 $\mu$m.

b) Bestimmung des Gehalts an Zuschlagstoffen im Mörtel:

iii) Ausschneiden eines Fragments des Zement-Kalk-Mörtels und dessen Trocknen bei einer Temperatur von nicht weniger als 40 °C für mindestens 8 Stunden, gefolgt von dessen vollständigem Schleifen, um Körner mit einer Abmessung von weniger als 0,125 mm zu erhalten.
iv) Bestimmung des Gehalts an Zuschlagstoffen im Zement-Kalk-Mörtel anhand der Menge des unlöslichen Rückstands in wässrigen HCl- und $Na_2CO_3$-Lösungen in der in Schritt iv) hergestellten Probe in Übereinstimmung mit der Norm PN-EN 196-2: 2013-11.

**dadurch gekennzeichnet, dass** es sich bei der mikroskopischen Untersuchung der hergestellten Probe um die Untersuchung der Bindemittelmasse in der Probe allein unter Vernachlässigung des Zuschlagskorns mit einem SEM-Rasterelektronenmikroskop, das mit einem EDS-Röntgendispersionsspektrometer integriert ist, handelt.

comprenant les étapes suivantes :

a) détermination du rapport atomique en % ciment/chaux dans le liant, comprenant :

i) collecte mécanique d'un fragment de mortier,
séchage de l'échantillon de l'étape i) à une température non inférieure à 40 °C pendant au moins 8 heures, puis immersion de l'échantillon refroidi dans une résine époxy sous vide, exposition de la surface de la section transversale du mortier par meulage et polissage, ainsi que pulvérisation d'un matériau conducteur choisi dans un groupe comprenant l'or, le platine, le palladium, l'osmium et le carbone, l'épaisseur de la couche formée par pulvérisation étant d'au moins 1 nm ;
iii) détermination du rapport ciment/chaux dans la masse de liant sur la base du rapport Si/Ca, par examen microscopique de l'échantillon de l'étape ii) à l'aide d'un microscope à balayage ayant une tension d'accélération d'au moins 10 kV et une ouverture d'au moins 1 $\mu$m

b) détermination de la teneur en agrégats dans le mortier :

iii) découpage d'un fragment de mortier de ciment et de chaux et son séchage à une température non inférieure à 40 °C pendant au moins 8 heures, suivi de son broyage complet pour obtenir des grains d'un diamètre inférieur à 0,125 mm
iv) détermination de la teneur en agrégats dans le mortier de ciment et de chaux sur la base de la quantité de résidus insolubles dans les solutions aqueuses de HCl et de $Na_2CO_3$ de l'échantillon préparé à l'étape iv), conformément à la norme PN-EN 196-2 : 2013-11.

**caractérisé en ce que** l'examen microscopique de l'échantillon préparé est l'examen de la masse de liant dans l'échantillon seul, en omettant le grain d'agrégat, à l'aide d'un microscope électronique à balayage SEM intégré à un spectromètre de dispersion d'énergie à rayons X EDS.

## Revendications

1. Un procédé pour déterminer la composition d'un mortier de maçonnerie à base de ciment et de chaux

FIG. 1

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **V. QUARZIONI et al.** *Construction and Building Materials*, 2006, vol. 20, 1069-1078
- **H. HE et al.** *Soil Dynamics and Earthquake Engineering*, 2016, vol. 89, 208-218
- **M. T. FREIRE et al.** *Construction and Building Materials*, 2019, vol. 196, 512-529